(19) Europäisches Patentamt
European Patent Office
Office européen des brevets

(11) Veröffentlichungsnummer: **0 058 975**
**B1**

# (12) EUROPÄISCHE PATENTSCHRIFT

(45) Veröffentlichungstag der Patentschrift: **12.12.84**

(51) Int. Cl.³: **C 07 D 401/14**, A 61 K 31/445

(21) Anmeldenummer: **82101315.8**

(22) Anmeldetag: **20.02.82**

(54) **Substituierte N-(4-Indolyl-piperidino-alkyl)-benzimidazolone, Verfahren zu ihrer Herstellung und ihre Verwendung als Pharmazeutika.**

(30) Priorität: **25.02.81 US 237966**

(43) Veröffentlichungstag der Anmeldung: **01.09.82 Patentblatt 82/35**

(45) Bekanntmachung des Hinweises auf die Patenterteilung: **12.12.84 Patentblatt 84/50**

(84) Benannte Vertragsstaaten: **AT BE CH DE FR IT LI LU NL SE**

(56) Entgegenhaltungen:
**EP - A - 0 030 174**
**DE - A - 2 609 289**
**US - A - 4 254 127**

(73) Patentinhaber: **Boehringer Ingelheim Ltd., 90 East Ridge P.O. Box 368, Ridgefield, Conn 06877 (US)**

(72) Erfinder: **Freter, Kurt, Dr., 103 Scodon Drive, Ridgefield/Conn. 06877 (US)**
Erfinder: **Fuchs, Viktor, 2 Cedar Lane, New Fairfield/Conn. 06810 (US)**
Erfinder: **Oliver, James T., Dr., 116 North Street, Middlebury/Conn. 06762 (US)**

(74) Vertreter: **Wehlmann, Gisela, Dipl.Chem. et al, BOEHRINGER INGELHEIM INTERNATIONAL GMBH ZA Patente Postfach 200, D-6507 Ingelheim am Rhein (DE)**

EP 0 058 975 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung bezieht sich auf neue substituierte N-(4-Indolyl-piperidino-alkyl)-benzimidazolone und deren nicht-toxische Säureadditionssalze, auf Verfahren zur Herstellung dieser Verbindungen, auf pharmazeutische Zusammensetzungen, die diese Verbindungen als aktive Bestandteile enthalten und auf ihre Anwendung als Antiallergika und Hypotensiva.

Beansprucht wird eine neue Klasse von Verbindungen der allgemeinen Formel

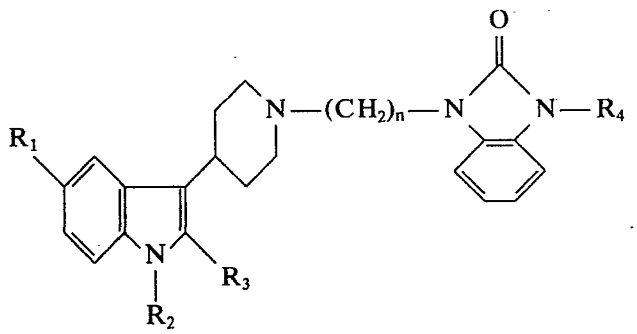

(I)

worin

$R_1$ Wasserstoff, Fluor, Chlor, Brom oder Methoxy;
$R_2$ Wasserstoff oder einen niederen Alkylrest;
$R_3$ Wasserstoff oder einen niederen Alkylrest;
$R_4$ Wasserstoff, einen niederen Alkylrest oder einen Alkenylrest mit 3 Kohlenstoffatomen und
n eine der Zahlen 2, 3, 4, 5 oder 6 bedeutet und deren physiologisch verträgliche Säureadditionssalze.

Der Ausdruck »niederes Alkyl« bezeichnet eine Alkylgruppe von 1–3 Kohlenstoffatomen, vorzugsweise die Methylgruppe.

Die Verbindungen der allgemeinen Formel I können nach verschiedenen an sich bekannten Methoden hergestellt werden, wobei die folgenden Verfahren bevorzugt sind:

Verfahren A

Durch Alkylierung eines 3-(1,2,5,6-Tetrahydro-4-pyridyl)-indols der allgemeinen Formel

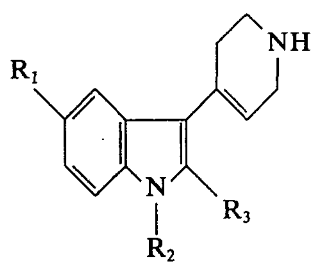

(II)

worin $R_1$, $R_2$ und $R_3$ die oben angegebene Bedeutung besitzen, mit einem N-($\omega$-Halogenalkyl)-benzimidazolon der allgemeinen Formel

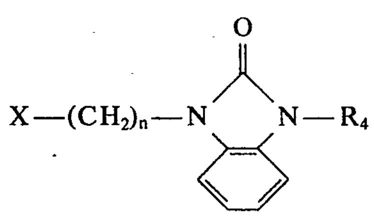

(III)

worin $R_4$ und n die oben angegebene Bedeutung besitzen und X ein Chlor-, Brom- oder Jodatom bedeutet, und Hydrierung des so erhaltenen Zwischenprodukts der allgemeinen Formel

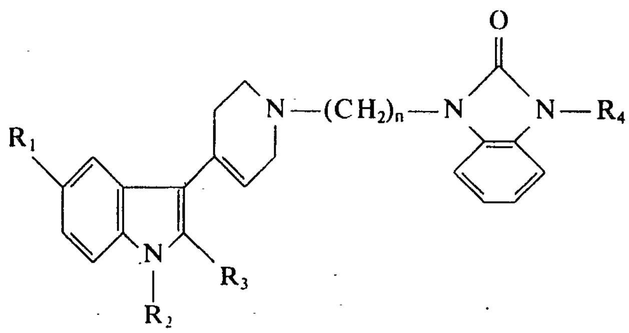

(IV)

mit Wasserstoff in Gegenwart eines Edelmetallkatalysators.

Die Alkylierung wird in Gegenwart eines inerten polaren oder unpolaren organischen Lösungsmittels, z. B. Äthanol, Dimethylformamid, Tetrahydrofuran usw., und vorzugsweise in Gegenwart eines anorganischen oder organischen säurebindenden Mittels wie Natriumhydroxyd, Natriumcarbonat, Natriumbicarbonat, Triäthylamin oder dergleichen bei Temperaturen zwischen 20°C und der Siedetemperatur des Lösungsmittels durchgeführt.

Die nachfolgende Hydrierung wird bei Temperaturen um 20°C bei normalem oder erhöhtem Druck durchgeführt. Als geeignete Edelmetallkatalysatoren seien Palladium und Platin genannt.

Verfahren B

Durch Alkylierung eines 3-(4-Piperidyl)-indols der allgemeinen Formel

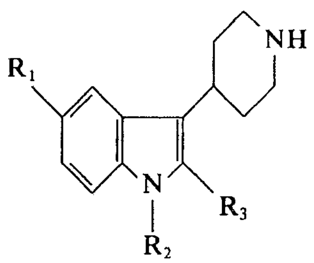

(V)

worin $R_1$, $R_2$ und $R_3$ die oben angegebene Bedeutung haben, mit einem N-($\omega$-Halogenalkyl)-benzimidazolon der allgemeinen Formel III.

Die Alkylierung wird in Gegenwart eines inerten polaren oder unpolaren organischen Lösungsmittels wie Äthanol, Dimethylformamid, Tetrahydrofuran usw., und vorzugsweise in Gegenwart eines anorganischen oder organischen säurebindenden Mittels wie Natriumhydroxiyd, Natriumcarbonat, Natriumbicarbonat, Triäthylamin und dergleichen bei Temperaturen zwischen 20°C und der Siedetemperatur des Lösungsmittels durchgeführt.

Verfahren C

Durch Alkylierung der Verbindung 4-Piperidon der Formel

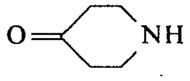

(VI)

mit einem N-($\omega$-Halogenalkyl)-benzimidazolon der allgemeinen Formel III, wobei das auf diese Weise erhaltene Zwischenprodukt der allgemeinen Formel

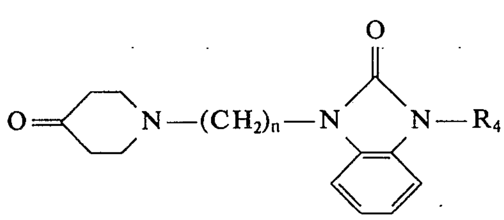

(VII)

worin $R_4$ und n die oben angegebene Bedeutung haben, in saurer Lösung mit einem Indol der allgemeinen Formel

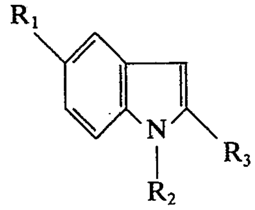

(VIII)

worin $R_1$, $R_2$ und $R_3$ die oben angegebene Bedeutung haben, zum entsprechenden Zwischenprodukt der Formel IV umgesetzt wird, und wobei das Zwischenprodukt der Formel IV, wie in Verfahren A beschrieben, hydriert wird.

Die Reaktion des Zwischenproduktes VII mit dem Indol der Formel VIII erfolgt in wäßriger saurer Lösung bei Zimmertemperatur.

Verfahren D

Durch Alkylierung eines 3-(4-Piperidyl)-indols der allgemeinen Formel V mit einem $\alpha$, $\omega$-Dihalogenalkan der allgemeinen Formel

$$X—(CH_2)_n—X \qquad (IX)$$

worin n die oben angegebene Bedeutung besitzt und die Reste X, die gleich oder verschieden sein können, eines der Halogenatome Chlor, Brom oder Jod bedeuten, und Alkylierung des auf diese Weise erhaltenen Zwischenproduktes der allgemeinen Formel

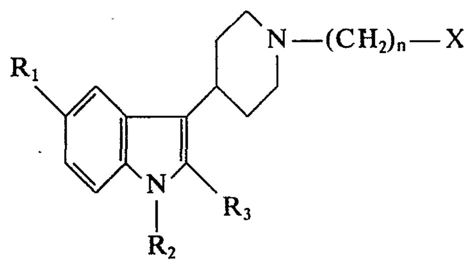

(X)

worin $R_1$, $R_2$, $R_3$, n und X die oben angegebene Bedeutung besitzen, mit einem Benzimidazolon der allgemeinen Formel

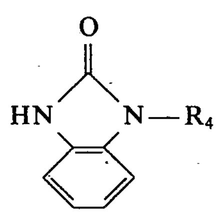

(XI)

worin $R_4$ die oben angegebene Bedeutung besitzt.

Die Verbindungen der allgemeinen Formel I sind basisch und bilden daher Additionssalze mit anorganischen oder organischen Säuren. Physiologisch verträgliche Säureadditionssalze können beispielsweise gebildet werden mit Halogenwasserstoffsäuren, vorzugsweise Chlorwasserstoffsäure oder Bromwasserstoffsäure, Salpetersäure, Schwefelsäure, o-Phosphorsäure, Weinsäure, Zitronensäure, Maleinsäure, Fumarsäure, Propionsäure, Buttersäure, Essigsäure, Bernsteinsäure, Methansulfonsäure, Benzolsulfonsäure, p-Toluolsulfonsäure usw..

Die Ausgangsverbindungen für die Verfahren A bis D sind bekannt oder können nach an sich bekannten Methoden hergestellt werden.

Verbindungen der Formel II und Verfahren zu ihrer Herstellung werden beschrieben von K. Freter in J. Org. Chem. 40, 2525 (1975).

Verbindungen der Formel III und XI beschreiben F. Awouters et al. in »Drugs Affecting the Respiratory System«, ACS Symposium Series 118, page 179 (1980).

Verbindungen der allgemeinen Formel V und Verfahren zu ihrer Herstellung werden in Helv. Chim. Acta 51, 260 (1968) von D. Beck et al. beschrieben.

Verbindungen der allgemeinen Formeln VI und VIII können von der Firma Aldrich Chemical Co., Inc., Milwaukee, Wisconsin, bezogen werden.

Die Verbindungen der vorliegenden Erfindung und ihre physiologisch verträglichen Säureadditionssalze besitzen wertvolle pharmakologische Eigenschaften. In warmblütigen Tieren, z.B. Ratten, besitzen sie eine antiallergische Wirksamkeit und können daher zur Behandlung von allergischen Erkrankungen wie allergischem Asthma, Rhinitis, Conjunctivitis, Heufieber, Nesselfieber, Lebensmittelallergien und ähnlichem verwendet werden.

Die pharmakologische Untersuchung der neuen Verbindungen zeigte, daß hier zwei biologische Mechanismen wirken: Erstens stabilisieren die Verbindungen die Mastzellen und verhindern so die normalerweise als Folge der Reaktion Antigen-Antikörper oder anderer Stimulantien stattfindende Freisetzung der Überträgersubstanzen. Zweitens besitzen sie Antihistamin-Eigenschaften, d.h. sie verhindern die Wirkung des Histamins, des Hauptauslösers menschlicher Allergien. Die erstgenannte Eigenschaft wurde durch die Mastzellen-Stabilisierungsuntersuchungen festgestellt, die zweite Eigenschaft in Untersuchungen auf Antihistaminwirkung und beide zusammen zeigen sich im PCA (Passive Cutaneous Anaphylaxis)-Test.

a) PCA-Test an Ratten

Es wurde im wesentlichen die Versuchsanordnung verwendet, die bei J. El-Azab und P. B. Stewart, »Pharmacological Profile of a New Anti-Allergic Compound PRD-92-EA.« Int. Archs. Allergy Appl. Immunol. 55: 350—361, 1977, beschrieben ist.

Eine Verdünnung von Antieialbumin-IgE-Antiserum wurde zur Erreichung reproduzierbarer Hautreaktionen mit Durchmessern zwischen 10 und 15 mm in nichtsensibilisierten Ratten verwendet. 0,1 ml dieser Antiserumverdünnung wurde männlichen CD-Ratten mit einem Gewicht zwischen 150 und 160 g auf jede Seite des rasierten Rückens intradermal injiziert, und zwar vor dem Antigenreiz. Die Testverbindungen wurden in Wasser gelöst, mit 0,5 ml einer Lösung von 5 mg Eialbumin und 2% Evans Blue gemischt und den Tieren 1 ml/kg dieser Mischung 24 Stunden nach der passiven Sensibilisierung i.v. injiziert.

Für die Untersuchung auf orale Wirksamkeit wurden die Testverbindungen in einer 1%igen Akaziengummilösung suspendiert und mittels einer Schlundsonde den Tieren 1 ml/kg verabreicht. 20 bis 30 Minuten nach der oralen Gabe der Testsubstanzen erfolgte ein Antigenreiz durch Gabe von 0,015 mg Eialbumin in 0,5 ml einer 2%igen Evans Blue-Lösung. 15 Minuten nach dem Antigenreiz wurde zur Erzeugung einer starken Antihistaminwirkung eine intradermale Injektion von 3 µg/0,1 ml Histamin in physiologischer Kochsalzlösung gegeben.

30 Minuten nach dem Antigenreiz wurden sowohl die i.v. als auch die oral behandelten Ratten mittels $CO_2$ getötet. Entlang der Wirbelsäure wurde ein Schnitt gemacht, die Haut zurückgeschlagen und der Durchmesser der blau gefärbten Stellen in Millimetern gemessen. Die mittlere Fläche wird für jeden Flecken in Quadratmillimetern bestimmt und daraus die mittlere Gesamtfläche für diese Testgruppe errechnet. Die mittlere Fläche einer unbehandelten Kontrollgruppe in Quadratmillimetern wurde mit 100% bezeichnet und das Resultat der Testverbindungen in Prozentänderung gegenüber dem Kontrollwert ausgedrückt. Als $ED_{50}$ wurde eine 50%ige Reduktion der Fläche bezeichnet; sie wurde bestimmt nach der Methode von Litchfield, J. T. Jr. and Wilcoxon, F. »A simplified Method of evaluating Dose-Effect Experiments«, J. Pharmacol. Exp. Therap. 96: 99—113, 1949.

b) In-Vivo-Hemmung der Histamin-induzierten Blaufärbung der Rattenhaut

Männliche CD-Ratten mit einem Gewicht zwischen 150 und 160 g wurden in zwei Gruppen eingeteilt. Beiden Gruppen wurde das Haar am Rücken entfernt. Eine unbehandelte Kontrollgruppe erhielt 10 ml/kg normale physiologische Kochsalzlösung in 1%iger Akaziengummilösung p.o. Die zum Test herangezogene Gruppe erhielt 10 ml/kg Testsubstanz, suspendiert in 1%iger Akaziengummilösung, ebenfalls oral. Alle Tiere erhielten außerdem 0,5 ml 2%iger Evans Blue-Lösung in normaler physiologischer Kochsalzlösung i.v. 20 Minuten nach Gabe der Testverbindung oder des Vehikels wurde in beide Seiten der rasierten Rückenhaut der Ratten 0,1 ml einer Lösung von 3 µg Histamindiphosphat in normaler physiologischer Kochsalzlösung intradermal injiziert. 15 Minuten nach der Histamininjektion wurden alle Ratten mittels $CO_2$ getötet.

Entlang der Wirbelsäule wurde ein Schnitt gelegt und die Haut sorgfältig abgetrennt. Die Dorsalhaut wurde zurückgeschlagen und die Durchmesser der blau gefärbten Flecken gemessen. Für jeden blauen Flecken wurde die Fläche in Quadratmillimetern bestimmt und die mittlere Fläche für die Kontroll- und die Testgruppen errechnet. Die mittlere Fläche der Kontrollgruppe wurde mit 100% bezeichnet. Die Resultate der Testgruppe wurden ausgedrückt als Prozentänderung gegenüber der Kontrollgruppe. Die $ED_{50}$ (d.h., die 50%ige Reduktion der blau gefärbten Fläche) wurde durch lineare Regressionsanalyse bestimmt.

c) Inhibierung der peritonealen Mastzellendegranulation (MCD) in mit Eialbuminantiserum oder durch Induktion von N-Methyl-homoanisylamin-formaldehyd-copolymer passiv sensibilisierten Ratten

Dieser Test wurde entlehnt von I. Mota und A. G. Osler »Mast Cell Degranulation«, Methods in Medical Research (ed. H. H. Eisen), Yearbook Medical Publication, Chicago, 1964.

Männliche CD-Ratten mit einem Gewicht zwischen 150 und 160 g wurden in fünf Gruppen eingeteilt:

Gruppe I: nicht spezifische MCD-Kontrolle (3 Ratten)
Gruppe II: positive MCD-Kontrolle (5 Ratten)
Gruppen III, IV, V: positive MCD nach Testsubstanz (jeweils 5 Ratten).

Gruppe I erhielt 3 ml normales Rattenserum i.p. Die Gruppen II bis V erhielten 3 ml eines Antiserums, das eine 60 bis 80% höhere Degranulation bewirkt als normales Rattenserum, ebenfalls i.p. 18 bis 24 Stunden später wurde die jeweilige Testverbindung verabreicht. Dies geschah bei i.v.-Gabe sofort, bei i.p.-Gabe 5 Minuten und bei p.o.-Gabe 20 Minuten vor einem i.p.-verabfolgten Antigenreiz mittels 0,5 mg/kg von zweimal kristallisiertem Eialbumin in einer Konzentration von 0,005% physiologischer Kochsalzlösung. 15 Minuten nach dem Reiz wurden die Ratten mittels $CO_2$ getötet. Falls zur Induktion der Degranulation der Mastzellen N-Methyl-homoanisylamin-formaldehyd-copolymer verwendet wurde, erhielt die Gruppe I 3 ml Hank's-Lösung mit einem pH-Wert von 7,2 bis 7,4 i.p. verabreicht.

Die positive Kontrollgruppe und die Gruppen, die die Testverbindungen erhielten, wurden mit 20 μg/kg der oben genannten Verbindung in 3 ml Hank's-Lösung bei pH 7,2 bis 7,4 i.p. behandelt. Auch diese Ratten wurden 5 Minuten nach der i.p.-Injektion mittels $Co_2$ getötet.

Um das Ausmaß der Degranulation der Mastzellen in den unbehandelten und den behandelten Gruppen von Ratten zu bestimmen, wurden Mikrotomschnitte des Mesenteriums hergestellt und analog der von A. Fügner angegebenen Methode »An Improved Method for the Study of Reaginmediated Mast Cell Degranulation in Rats«, Experientia, 29: 708, 1973, analysiert. Die Resultate wurden berechnet als Prozent Inhibierung der Degranulation wie folgt:

$$\text{Prozent Inhibierung} = 100 - \left[\frac{\text{Experiment} - \text{negative Kontrolle}}{\text{positive Kontrolle} - \text{negative Kontrolle}}\right] \times 100.$$

Die folgende Tabelle zeigt die mit den angeführten Test erhaltenen Resultate für die Verbindung des Beispiels 4 im Vergleich mit Oxatomid, einer bekannten Verbindung gleicher Wirkungsrichtung.

| Verbindung | $ED_{50}$ mg/kg | | |
|---|---|---|---|
| | PCA | MCD | Antihist. |
| Oxatomid | 8,3 | neg. | 8,1 |
| Verbindung Beispiel 4 | 1,6 | 3,0–6,4 | 8,7 |

Diese Resultate zeigen, daß die Verbindung des Beispiels 4 in zweierlei Hinsicht überlegen ist; sie besitzt eine bemerkenswerte Mastzellen stabilisierende Wirkung neben guten Antihistamineigenschaften und ist fünfmal stärker wirksam als die bekannte Verbindung Oxatomid.

Die folgenden Beispiele dienen zur Erläuterung der vorliegenden Erfindung, ohne sie jedoch zu beschränken:

## Beispiel 1

### N-{3-[4-(2-Methyl-3-indolyl)-piperidino]-propyl}-benzimidazolon und dessen Hydrochlorid nach Verfahren A

Eine Mischung aus 1,06 g 2-Methyl-3-(1,2,5,6-tetrahydropyridyl-4)-indol, 1,05 g N-(3-Chlorpropyl)-benzimidazolon, 0,42 g Natriumbicarbonat, 20 ml Dimethylformamid und 20 ml Tetrahydrofuran werden 18 Stunden unter Rühren auf 100°C erhitzt. Danach wird das Reaktionsgemisch in eine Mischung aus 200 g Eis und 10 ml konzentriertem Ammoniak gegossen, der sich dabei bildende Niederschlag abfiltriert und aus Äthanol umkristallisiert.

Man erhält 1,2 g (62% d.Th.) des Zwischenprodukts der Formel IV ($R_1$ = H, $R_2$ = H, $R_3$ = $-CH_3$, n = 3, $R_4$ = H) mit einem Fp. von 215°C.

1,5 g des Zwischenproduktes werden in 100 ml Essigsäure gelöst und 24 Stunden mit 0,8 g Palladium (5% auf Teerkohle) bei 20°C in einer Wasserstoffatmosphäre und unter 5 atü Druck geschüttelt. Danach wird der Katalysator abfiltriert und das Filtrat in eine Mischung von Eis und Ammoniak gegossen, wobei die gewünschte freie Base ausfällt. Der Niederschlag wird abfiltriert, getrocknet, in Äthanol gelöst und durch Zugabe ätherischer Salzsäure in das Hydrochlorid verwandelt.

Man erhält 1,05 g (70% d.Th.) der Titelverbindung vom Fp. 264—269°C.

Beispiel 2

N-{4-[4-(3-Indolyl)-piperidono]-butyl}-benzimidazolon nach Verfahren B

Eine Mischung aus 3 g 4-(3-Indolyl)-piperidin, 4 g 1-(4-Chlor-butyl)-3-isopropenyl-benzimidazolon, 1,3 g Natriumbicarbonat, 30 ml Dimethylformamid und 30 ml Tetrahydrofuran werden 16 Stunden unter Rückfluß erhitzt. Darauf wird das Reaktionsgemisch in eine Mischung aus Eis und Ammoniak gegossen, die Mischung mit Äthylacetat extrahiert und die Extraktionslösung mit Wasser gewaschen, getrocknet und im Vakuum zur Trockne verdampft. Der Rückstand wird in Äther gelöst, mit ätherischer Salzsäure versetzt und der ausfallende Niederschlag filtriert, getrocknet und in 100 ml Äthanol gelöst. Die erhaltene Lösung wird gekühlt, unter sorgfältigem Rühren mit 16 ml konzentrierter Schwefelsäure versetzt und 2 Stunden bei 20°C stehengelassen. Sodann wird das Gemisch in eine Mischung von Eis und Ammoniak gegossen, der sich bildende Niederschlag abfiltriert und aus Äthanol umkristallisiert.

Man erhält 3,2 g (55% d.Th.) der Titelverbindung vom Fp. 196°C.

Beispiel 3

N-{3-[4-(1-Methyl-3-indolyl)-piperidino]-propyl}-benzimidazolon nach Verfahren C

Ein Gemisch von 6,3 g N-(3-Chlor-propyl)-benzimidazolon, 4,6 g 4-Piperidin-hydrochloridmonohydrat, 5,0 g Natriumbicarbonat, 50 ml Tetrahydrofuran und 50 ml Dimethylformamid werden 36 Stunden unter Rückfluß erhitzt. Das erhaltene Reaktionsgemisch wird wie üblich aufgearbeitet, und man erhält 3,2 g (39% d.Th.) N-[3-(4-Oxo-piperidino)-propyl]-benzimidazolon vom Fp. 134—136°C.

Eine Mischung, bestehend aus 2,7 g N-[3-(4-Oxo-piperidino)-propyl]-benzimidazolon, 1,3 g 1-Methylindol, 40 ml Essigsäure und 10 ml 2N Phosphonsäure werden 6 Tage bei 20°C stehengelassen. Danach wird das Reaktionsgemisch in eine Mischung von Eis und Ammoniak gegossen, die wäßrige Mischung mit Äthylacetat extrahiert und die Extraktionslösung getrocknet und im Vakuum zur Trockne verdampft. Der Rückstand wird durch Chromatographie an Kieselsäure gereinigt, wobei als Laufmittel Methylenchlorid/Methanol/Ammoniak im Verhältnis 90:9:1 benutzt werden. Die Hauptfraktion wird ohne vorherige Isolierung, wie in Beispiel 1 beschrieben, hydriert und das Endprodukt aus Äthanol umkristallisiert.

Man erhält 2,4 g (62% d.Th.) der Titelverbindung vom Fp. 180°C.

Beispiel 4

Analog der in Beispiel 1 beschriebenen Methode erhält man aus 3-)1,2,3,4-Tetrahydropyridyl-4)-indol und N-(3-Chlor-propyl)-benzimidazolon das N-{3-[4-(3-Indolyl)-piperidino]-propyl}-benzimidazolon-hydrochlorid vom Fp. 204°C.

Beispiel 5

Analog dem im Beispiel 2 beschriebenen Verfahren erhält man aus 4-(5-Methoxy-3-indolyl)-piperidin und N-(3-Chlor-propyl)-benzimidazolon das N-{3-[4-(5-Methoxy-3-indolyl)-piperidino]-propyl}-benzimidazolon-hydrochlorid vom Fp. 178°C.

Beispiel 6

Analog dem in Beispiel 2 beschriebenen Verfahren erhält man aus 4-(3-Indolyl)-piperidin und N-(3-Chlor-propyl)-N'-isopropenyl-benzimidazolon das N-{3-[4-(3-Indolyl)-piperidino]-propyl}-N'-isopropenyl-benzimidazolon vom Fp. 68°C.

Beispiel 7

Analog der in Beispiel 2 beschriebenen Methode wird aus 4-(1-Isopropyl-3-indolyl)-piperidin und N-(3-Chlor-propyl)-benzimidazolon das N-{3-[4-(1-Isopropyl-3-indolyl)-piperidino]-propyl}-benzimidazolon-hydrochlorid vom Fp. 145°C hergestellt.

Beispiel 8

Analog Beispiel 2 erhält man aus 4-(3-Indolyl)-piperidin und N-(2-Chlor-äthyl)-benzimidazolon das N-{2-[4-(3-Indolyl)-piperidino]-äthyl}-benzimidazolon vom Fp. 116°C.

Beispiel 9

Analog Beispiel 2 erhält man aus 4-(3-Indolyl)-piperidin und N-(3-Chlor-propyl)-N'-methyl-benzimidazolon das N-{3-[4-(3-Indolyl)-piperidino]-propyl}-N'-methyl-benzimidazolon-hydrochlorid vom Fp. 140°C.

Beispiel 10

Analog Beispiel 3 erhält man aus N-[3-(4-Oxopiperidino)-propyl]-benzimidazolon und 1-Propylindol das N-{3-[4-(1-Propyl-3-indolyl)-piperidino]-propyl}-benzimidazolon vom Fp. 103°C.

Beispiel 11

Analog Beispiel 2 erhält man aus 4-(2-Methyl-5-chlor-3-indolyl)-piperidin und N-(3-Chlor-propyl)-benzimidazolon das N-{3-[4-(2-Methyl-5-chlor-3-indolyl)-piperidino]-propyl}-benzimidazolon vom Fp. 124°C.

Beispiel 12

Analog Beispiel 2 erhält man aus 4-(3-Indolyl)-piperidin und N-(5-Brom-pentyl)-benzimidazolon (Fp. 72—75°C) das N-{5-[4-(3-Indolyl)-piperidino]-pentyl}-benzimidazolon vom Fp. 137—140°C.

Beispiel 13

Analog Beispiel 2 erhält man aus 4-(3-Indolyl)-piperidin und N-(6-Brom-hexyl)-benzimidazolon (Fp. 103—105°C) das N-{6-[4-(3-Indolyl)-piperidino]-hexyl}-benzimidazolon vom Fp. 127—130°C.

Beispiel 14

N-{3-[4-(3-Indolyl)-piperidino]-propyl}-N'-isopropenyl-benzimidazolon nach Verfahren D

Eine Mischung von 2 g 4-(3-Indolyl)-piperidin (Formel V: $R_1$, $R_2$, $R_3$ = H) und 20 ml 1-Brom-3-chlorpropan werden 72 Stunden bei Raumtemperatur gerührt. Das Gemisch wird dann mit 1 N Salzsäure extrahiert, der Extrakt mit Natriumcarbonat leicht basisch gemacht und mit Essigester extrahiert. Der Rückstand, 1-Chlor-propyl-4-(3-indolyl)-piperidin, wird nach Trocknen und Verdampfen des Lösungsmittels ohne weitere Reinigung für die nächste Stufe verwendet.

0,5 g des Zwischenproduktes werden in 5 ml Tetrahydrofuran gelöst und zu einer gekühlten Suspension von N-Isopropenyl-benzimidazolon-Natriumsalz (erhalten aus 0,34 g N-Isopropenyl-benzimidazolon und 0,05 g Natriumhydrid) in 5 ml Dimethylformamid zugegeben. Die Mischung wird 16 Stunden bei Raumtemperatur gerührt und dann in ein Gemisch aus Eiswasser und Ammoniak gegossen. Die sich abscheidenden Kristalle werden abfiltriert und getrocknet.

Man erhält 0,65 g (81% d.Th.) der Titelverbindung vom Fp. 68°C.

Für die pharmazeutische Verwendung können die erfindungsgemäßen Verbindungen warmblütigen Tieren topisch, peroral, parenteral oder durch Inhalation verabreicht werden. Die Verbindungen liegen hierbei als aktive Bestandteile in üblichen Darreichungsformen vor, z.B. in Zusammensetzungen, die im wesentlichen aus einem inerten pharmazeutischen Träger und einer effektiven Dosis des Wirkstoffes bestehen, wie z.B. Tabletten, Dragees, Kapseln, Oblaten, Pulver, Lösungen, Suspensionen,

Inhalationsaerosole, Salben, Emulsionen, Sirupe, Suppositorien usw. Eine Wirkdosis der erfindungsgemäßen Verbindungen umfaßt 0,013 bis 0,26 mg/kg Körpergewicht.

Nachfolgend werden Beispiele für einige pharmazeutische Zusammensetzungen unter Verwendung einer erfindungsgemäßen Verbindung als aktiver Bestandteil angegeben. Falls nicht ausdrücklich anders bezeichnet, handelt es sich bei den Teilen um Gewichtsteile.

Beispiel 15

Tabletten

Die Tablette enthält folgende Bestandteile:

| | | |
|---|---|---|
| N-{3-[4-(3-Indolyl)-piperidino]-propyl}-benzimidazolon | | 0,010 Teile |
| Stearinsäure | | 0,010 Teile |
| Dextrose | | 1,890 Teile |
| | gesamt | 1,910 Teile |

Herstellung

Die Stoffe werden in bekannter Weise zusammengemischt und die Mischung zu Tabletten verpreßt, von denen jede 1,91 g wiegt und 10 mg Wirkstoff enthält.

Beispiel 16

Salbe

Die Salbe setzt sich aus folgenden Bestandteilen zusammen:

| | | |
|---|---|---|
| N-{3-[4-(3-Indolyl)-piperidino]-propyl}-benzimidazolon | | 2,000 Teile |
| Rauchende Salzsäure | | 0,011 Teile |
| Natriumpyrosulfit | | 0,050 Teile |
| Mischung (1:1) von Cetylalkohol und Stearylalkohol | | 20,000 Teile |
| Weiße Vaseline | | 5,000 Teile |
| Synthetischen Bergamottöl | | 0,075 Teile |
| Destilliertes Wasser | ad | 100,000 Teile |

Herstellung

Die Bestandteile werden in an sich bekannter Weise innig zu einer Salbe vermischt, von der 100 g 2,0 g der Wirksubstanz enthalten.

Beispiel 17

Inhalationsaerosol

Das Aerosol ist aus folgenden Bestandteilen zusammengesetzt:

| | | |
|---|---|---|
| N-{3-[4-(3-Indolyl)-piperidino]-propyl}-benzimidazolon | | 1,00 Teile |
| Sojalecithin | | 0,20 Teile |
| Treibgasmischung (Frigen® 11, 12, und 14) | ad | 100,00 Teile |

Herstellung

Die Bestandteile werden in an sich bekannter Weise zusammengemischt und das Gemisch in Aerosolbehälter gefüllt, die ein Dosierventil enthalten, das pro Betätigung zwischen 5 und 20 mg Wirksubstanz abgibt.

Beispiel 18

Ampullenlösung

Die Lösung setzt sich aus folgenden Bestandteilen zusammen:

| | |
|---|---|
| N-{3-[4-(3-Indolyl)-piperidino]-propyl}-benzimidazolon · HCl | 5,0 Teile |
| Natriumpyrosulfit | 1,0 Teile |
| Äthylendiamintetraessigsäure, Natriumsalz | 0,5 Teile |
| Natriumchlorid | 8,5 Teile |
| Doppelt destilliertes Wasser | ad 1000,0 Teile |

Herstellung

Die einzelnen Bestandteile werden in einer genügenden Menge doppelt destillierten Wassers gelöst, die Lösung bis zur angegebenen Konzentration mit weiterem doppelt destilliertem Wasser verdünnt, die so erhaltene Lösung pyrogenfrei filtriert und das Filtrat unter aseptischen Bedingungen in 1 ml Ampullen abgefüllt, die anschließend sterilisiert und zugeschmolzen werden. Jede Ampulle enthält 5 mg des Wirkstoffs.

Anstelle der in den Beispielen 15 bis 18 genannten Wirkstoffe kann jede andere unter die allgemeine Formel I der vorliegenden Anmeldung fallende Verbindung beziehungsweise ein physiologisch verträgliches Säureadditionssalz einer solchen Verbindung verwendet werden. Auch kann die Menge des Wirkstoffs in den einzelnen Beispielen im oben angegebenen Dosisbereich variiert werden, und auch Menge und Art des inerten Trägerstoffs kann in weiten Grenzen variiert werden, wenn spezielle Anforderungen erfüllt werden müssen.

**Patentansprüche**

1. Verbindungen der allgemeinen Formel

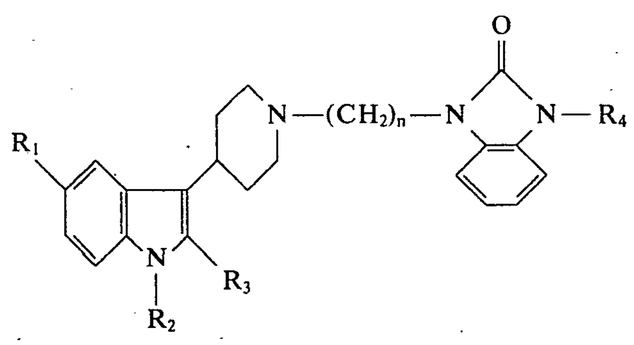

(I)

worin

$R_1$ Wasserstoff, Fluor, Chlor, Brom oder Methoxy;
$R_2$ Wasserstoff oder einen Alkylrest mit 1—3 Kohlenstoffatomen;
$R_3$ Wasserstoff oder einen Alkylrest mit 1—3 Kohlenstoffatomen;
$R_4$ Wasserstoff, einen Alkylrest mit 1—3 Kohlenstoffatomen oder den Isopropenylrest und
n eine der Zahlen 2, 3, 4, 5 oder 6 bedeuten sowie deren physiologisch verträgliche Säureadditionssalze.

2. N-{3-[4-(3-Indolyl)-piperidino]-propyl}-benzimidazolon und dessen physiologisch verträgliche Säureadditionssalze.

3. N-{4-[4-(3-Indolyl)-piperidino]-butyl}-benzimidazolon und dessen physiologisch verträgliche Säureadditionssalze.

4. Pharmazeutische Präparate, enthaltend als Wirkstoff eine oder mehrere Verbindungen der allgemeinen Formel I in Kombination mit üblichen Hilfs- und/oder Trägerstoffen.

5. Verfahren zur Herstellung von pharmazeutischen Präparaten gemäß Anspruch 4, dadurch gekennzeichnet, daß man Verbindungen der allgemeinen Formel I mit üblichen galenischen Hilfs- und/oder Trägerstoffen zu üblichen pharmazeutischen Anwendungsformen verarbeitet.

6. Verfahren zur Herstellung einer Verbindung der allgemeinen Formel I gemäß Anspruch 1 sowie von deren physiologisch verträglichen Säureadditionssalzen, dadurch gekennzeichnet, daß man

a) ein 3-(1,2,5,6-Tetrahydro-4-pyridyl)-indol der Formel

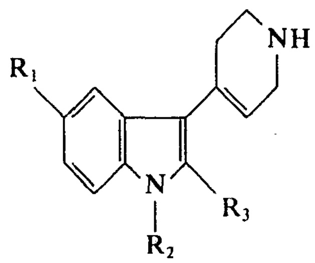

(II)

worin $R_1$, $R_2$ und $R_3$ die oben angegebene Bedeutung besitzen, mit einem N-($\omega$-Halogenalkyl)-benzimidazolon der allgemeinen Formel

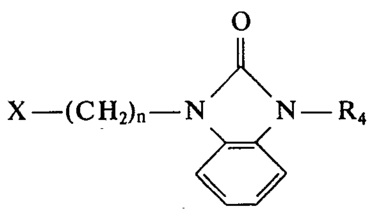

(III)

worin $R_4$ und n die oben angegebene Bedeutung besitzen und X ein Chlor-, Brom- oder Jodatom bedeutet, alkyliert und das so erhaltene Zwischenprodukt der allgemeinen Formel

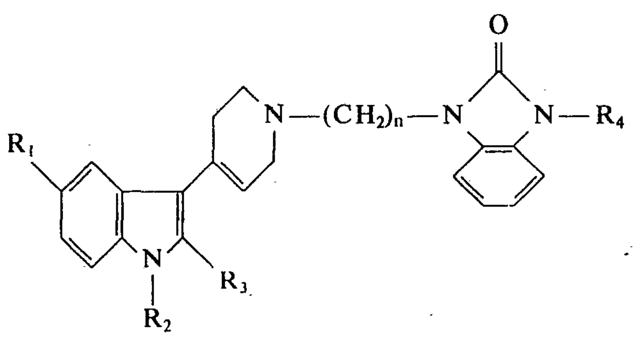

(IV)

worin $R_1$, $R_2$, $R_3$, $R_4$ und n die oben angegebene Bedeutung besitzen, mit Wasserstoff in Gegenwart eines Edelmetallkatalysators hydriert, oder daß man

b) ein 3-(4-Piperidyl)-indol der allgemeinen Formel

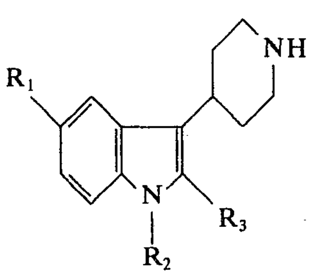

(V)

worin $R_1$, $R_2$ und $R_3$ die oben angegebene Bedeutung besitzen, mit einem N-($\omega$-Halogenalkyl)-benzimidazolon der allgemeinen Formel

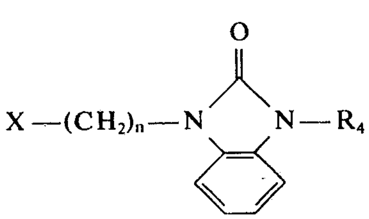

(III)

worin $R_4$ und n die oben angegebene Bedeutung besitzen und X ein Chlor-, Brom- oder Jodatom bedeutet, alkyliert, oder daß man

c) 4-Piperidon der Formel

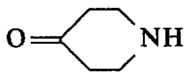

(VI)

mit einem N-(ω-Halogenalkyl)-benzimidazolon der allgemeinen Formel

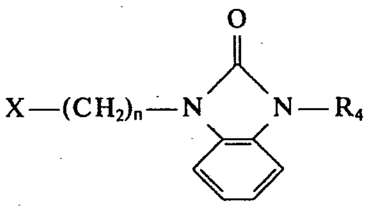

(III)

worin $R_4$ und n die oben angegebene Bedeutung besitzen und X ein Chlor-, Brom- oder Jodatom bedeutet, alkyliert und das so erhaltene Zwischenprodukt der allgemeinen Formel

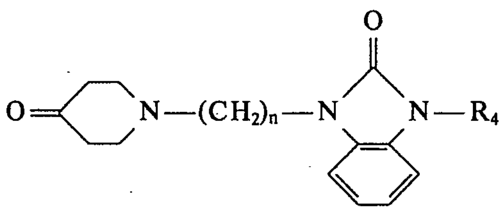

(VII)

worin $R_4$ und n die oben angegebene Bedeutung besitzen, in saurer Lösung mit einem Indol der allgemeinen Formel

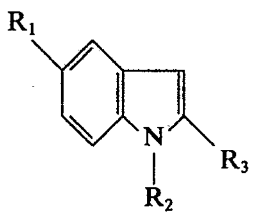

(VIII)

worin $R_1$, $R_2$ und $R_3$ die oben angegebene Bedeutung besitzen, zu einem weiteren Zwischenprodukt der allgemeinen Formel

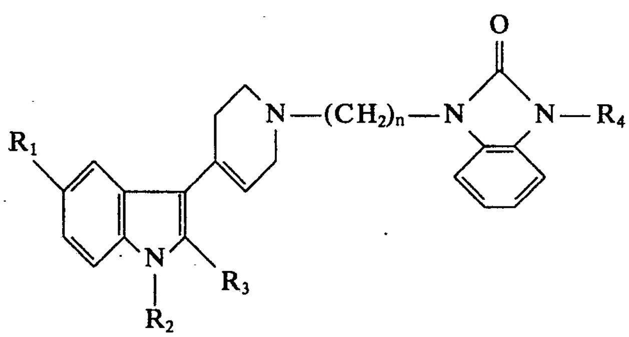

(IV)

worin $R_1$, $R_2$, $R_3$, $R_4$ und n die oben angegebene Bedeutung besitzen, umwandelt und die letztere Verbindung mit Wasserstoff in Gegenwart eines Edelmetallkatalysators hydriert, oder daß man

d) ein 3-(4-Piperidyl)-indol der allgemeinen Formel

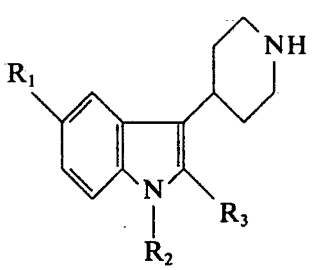

(V)

worin $R_1$, $R_2$ und $R_3$ die oben angegebene Bedeutung besitzen, mit einem $\alpha,\omega$-Dihalogenalkan der allgemeinen Formel

$$X—(CH_2)_n—X \qquad (IX)$$

worin n die oben angegebene Bedeutung besitzt und beide Reste X, die gleich oder verschieden sein können, ein Chlor-, Brom- oder Jodatom bedeuten, alkyliert und das so erhaltene Zwischenprodukt der allgemeinen Formel

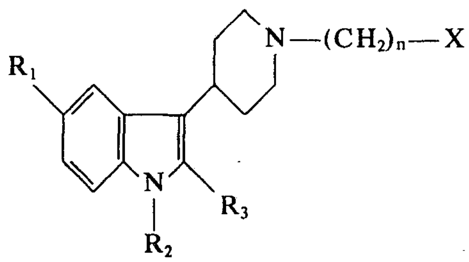

(X)

worin $R_1$, $R_2$, $R_3$, n und X die oben angegebene Bedeutung besitzen, mit einem Benzimidazolon der allgemeinen Formel

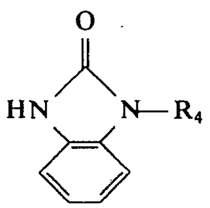

(XI)

worin $R_4$ die oben angegebene Bedeutung besitzt, alkyliert, und daß man gegebenenfalls ein nach einem der Verfahren A bis D hergestelltes Endprodukt der allgemeinen Formel I in an sich bekannter Weise in ein physiologisch verträgliches Säureadditionssalz überführt.

**Claims**

1. Compounds of general formula I

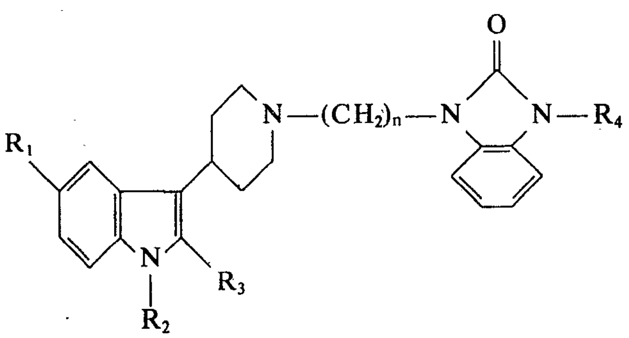

(I)

(wherein $R_1$ represents hydrogen, fluorine, chlorine, bromine or methoxy;

$R_2$ represents hydrogen or a $C_{1-3}$ alkyl group;
$R_3$ represents hydrogen or a $C_{1-3}$ alkyl group;
$R_4$ represents hydrogen, a $C_{1-3}$ alkyl group or an isopropenyl group; and
n is one of the numbers 2, 3, 4, 5 or 6)

and the physiologically acceptable acid addition salts thereof.

2. N-(3-[4-(3-Indolyl)-piperidino]-propyl)-benzimidazolone and the physiologically acceptable acid addition salts thereof.

3. N-(4-[4-(3-Indolyl)-piperidino]-butyl)-benzimidazolone and the physiologically acceptable acid addition salts thereof.

4. Pharmaceutical preparations containing as active substance one or more compounds of general formula I in conjunction with conventional excipients and/or carriers.

5. Process for preparing pharmaceutical preparations as claimed in claim 4, characterised in that compounds of general formula I are processed with conventional galenic excipients and/or carriers to produce conventional pharmaceutical forms for administration.

6. Process for preparing a compound of general formula I as claimed in claim 1 and the physiologically acceptable acid addition salts thereof, characterised in that

(a) a 3-(1,2,5,6-tetrahydro-4-pyridyl)-indole of formula

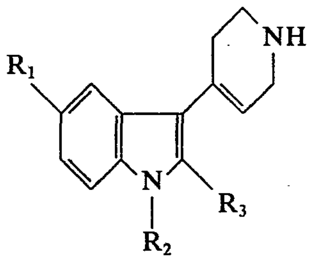

(II)

(wherein $R_1$, $R_2$ and $R_3$ are as hereinbefore defined) is alkylated with an N-($\omega$-haloalkyl)-benzimidazolone of general formula

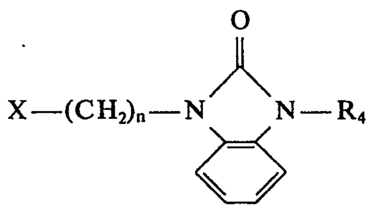

(III)

(wherein $R_4$ and n are as hereinbefore defined, and X represents a chlorine, bromine or iodine atom) and the resulting intermediate of general formula

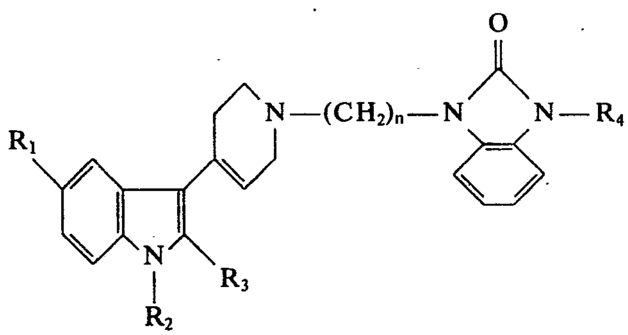

(IV)

(wherein $R_1$, $R_2$, $R_3$, $R_4$ and n are as hereinbefore defined) is hydrogenated with hydrogen in the presence of a noble metal catalyst; or

(b) a 3-(4-piperidyl)-indole of general formula

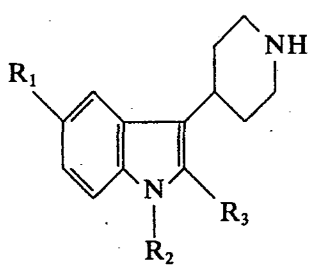

(V)

(wherein $R_1$, $R_2$ and $R_3$ are as hereinbefore definded) is alkylated with an N-($\omega$-haloalkyl)-benzimidazolone of general formula

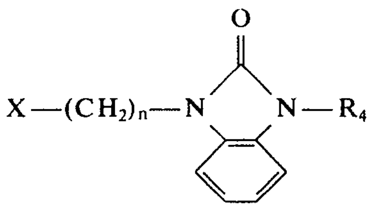

(III)

(wherein $R_4$ and n are as hereinbefore defined and X represents a chlorine, bromine or iodine atom); or

(c) 4-piperidone of formula

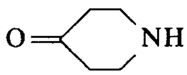

(VI)

is alkylated with an N-($\omega$-haloalkyl)-benzimidazolone of general formula

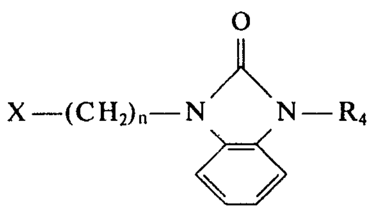

(III)

(wherein $R_4$ and n are as hereinbefore defined and X represents a chlorine, bromine or iodine atom) and the resulting intermediate of general formula

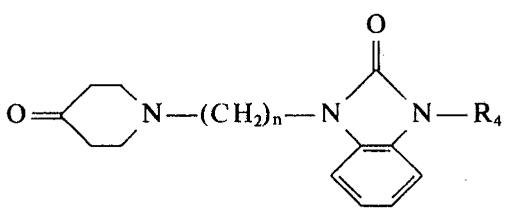

(VII)

(wherein $R_4$ and n are as hereinbefore defined) is reacted in acid solution with an indole of general formula

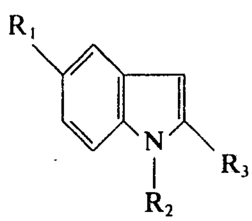

(VIII)

(wherein $R_1$, $R_2$ and $R_3$ are as hereinbefore defined) to form another intermediate of general formula

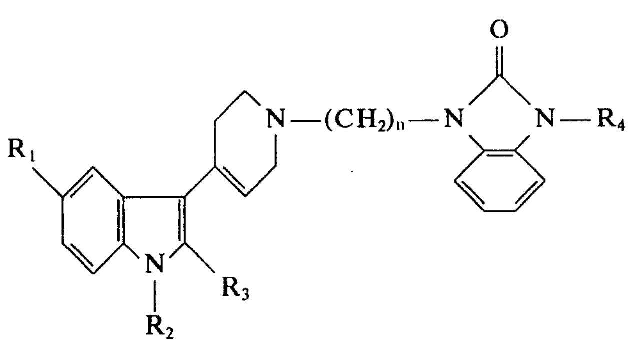

(IV)

(wherein $R_1$, $R_2$, $R_3$, $R_4$ and n are as hereinbefore defined) and the latter compound is hydrogenated with hydrogen in the presence of a noble netal catalyst; or

(d) a 3-(4-piperidyl)-indole of general formula

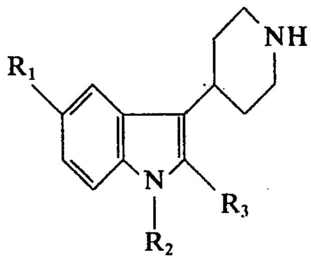

(V)

(wherein $R_1$, $R_2$ and $R_3$ are as hereinbefore defined) is alkylated with an $\alpha,\omega$-dihalo-alkane of formula

$$X—(CH_2)_n—X \quad (IX)$$

(wherein n is as hereinbefore defined and each X, which may be the same or different, represents a chlorine, bromine or iodine atom) and the resulting intermediate of general formula

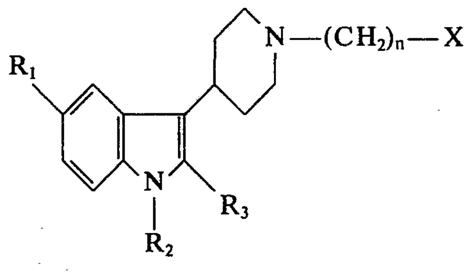

(X)

(wherein $R_1$, $R_2$, $R_3$, n and X are as hereinbefore defined) is alkylated with a benzimidazolone of general formula

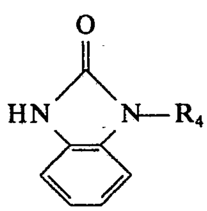

(XI)

(wherein $R_4$ is as hereinbefore defined); and if desired an end product of general formula I obtained by one of the processes A to D is converted into a physiologically acceptable acid addition salt thereof.

**Revendications**

1. Composés de formule générale:

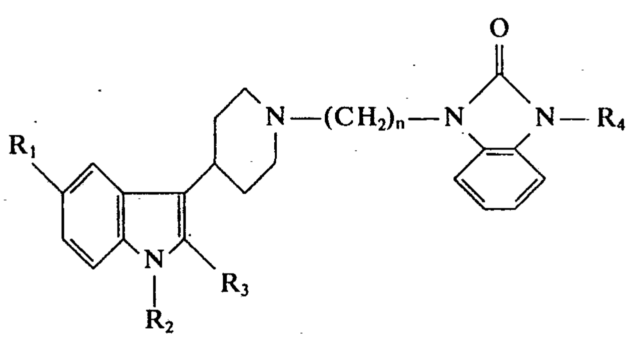

(I)

dans laquelle:

$R_1$ représente hydrogène, fluor, chlore, brome ou méthoxy;

$R_2$ représente hydrogène ou un radical alcoyle avec 1—3 atomes de carbone;
$R_3$ représente hydrogène ou un radical alcoyle avec 1—3 atomes de carbone;
$R_4$ représente hydrogène, un radical alcoyle avec 1—3 atomes de carbone ou le radical isopropényle et
n représente l'un des nombres 2, 3, 4, 5 ou 6 ainsi que leurs sels d'addition d'acides physiologiquement suppertables.

2. La N-{3-[4-(3-indolyl)-pipéridino]-propyl}-benzimidazolone et ses sels d'addition d'acides physiologiquement supportables.
3. La N-{4-[4-(3-indolyl)-pipéridino]-butyl}-benzimidazolone et ses sels d'addition d'acides physiologiquement supportables.
4. Préparations pharmaceutiques contenant en tant que substance active un ou plusieurs composés de formule générale I en combinaison avec des adjuvants et/ou excipients usuels.
5. Procédé pour la fabrication de préparations pharmaceutiques selon la revendication 4, caractérisé en ce qu'on transforme des composés de formule générale I au moyen d'adjuvants et/ou excipients galéniques usuels en des formes d'utilisation pharmaceutiques usuelles.
6. Procédé de préparation d'un composé de formule générale I selon la revendication 1 ainsi que de ses sels d'addition d'acides physiologiquement supportables, caractérisé en ce que:

a) on alcoyle un 3-(1,2,5,6-tétrahydro-4-pyridyl)-indole de formule:

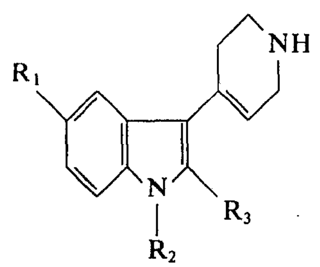

(II)

dans laquelle $R_1$, $R_2$ et $R_3$ possèdent la signification indiquée plus haut, au moyen d'une N-($\omega$-halogénoalcoyl)-benzimidazolone de formule générale:

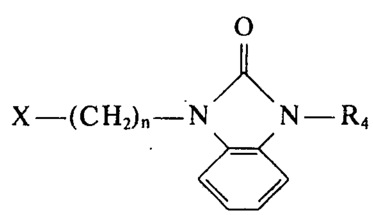

(III)

dans laquelle $R_4$ et n possèdent la signification indiquée plus haut et X représente un atome de chlore, de brome ou d'iode et en ce qu'on hydrogène le produit intermédiaire ainsi obtenu de formule générale:

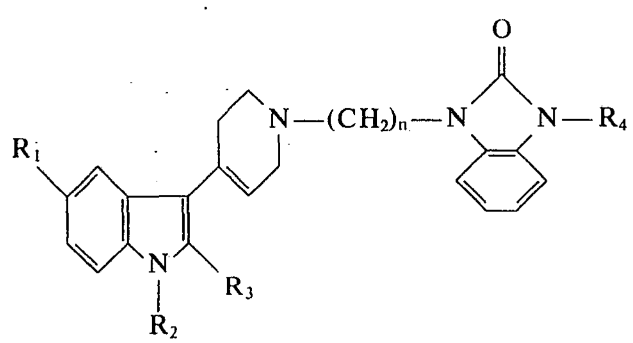

(IV)

dans laquelle $R_1$, $R_2$, $R_3$, $R_4$ et n possèdent la signification indiquée plus haut, au moyen d'hydrogène en présence d'un catalyseur à base de métal noble ou en ce que:

b) on alcoyle un 3-(4-pipéridyl)-indole de formule générale:

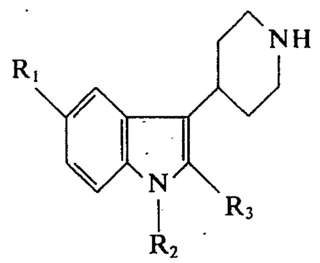

(V)

dans laquelle $R_1$, $R_2$ et $R_3$ possèdent la signification indiquée plus haut, au moyen d'une N-($\omega$-halogénoalcoyl)-benzimidazolone de formule générale:

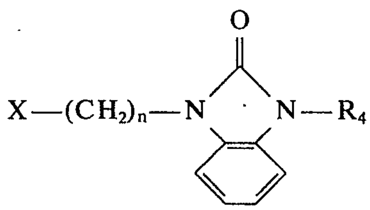

(III)

dans laquelle $R_4$ et n possèdent la signification indiquée plus haut et X représente un atome de chlore, de brome ou d'iode, ou en ce que:

c) on alcoyle la 4-pipéridone de formule:

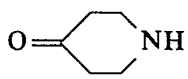

(VI)

au moyen d'une N-($\omega$-halogénoalcoyl)-benzimidazolone de formule générale:

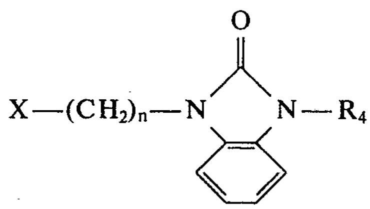

(III)

dans laquelle $R_4$ et n possèdent la signification indiquée plus haut et X représente un atome de chlore, de brome ou d'iode et en ce qu'on transforme le produit intermédiaire ainsi obtenu de formule générale:

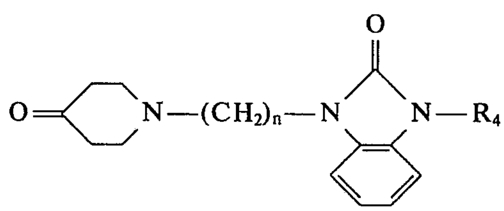

(VII)

dans laquelle $R_4$ et n possèdent la signification indiquée plus haut, en solution acide au moyen d'un indole de formule générale:

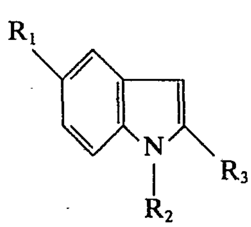

(VIII)

dans laquelle $R_1$, $R_2$ et $R_3$ possèdent la signification indiquée plus haut, en un autre compose intermédiaire de formule générale:

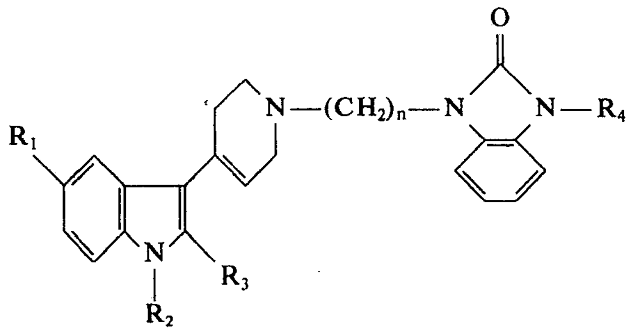

(IV)

dans laquelle $R_1$, $R_2$, $R_3$, $R_4$ et n possèdent la signification indiquée plus haut et en ce qu'on hydrogène le dernier composé au moyen d'hydrogène en présence d'un catalyseur à base de métal noble, ou en ce que:

d) on alcoyle un 3-(4-pipéridyl)-indole de formule générale:

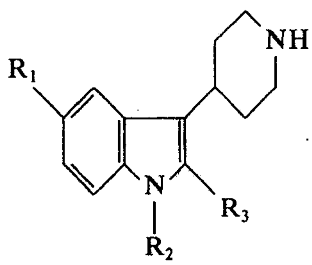

(V)

dans laquelle $R_1$, $R_2$ et $R_3$ possèdent la signification indiquée plus haut, au moyen d'un $\alpha,\omega$-dihalogénoalcane de formule générale:

$$X—(CH_2)_n—X \qquad (IX)$$

dans laquelle n possède la signification indiquée plus haut et les deux radicaux X, qui peuvent être identiques ou différents, représentent un atome de chlore, de brome ou d'iode, et en ce qu'on alcoyle le produit intermédiaire ainsi obtenu de formule générale:

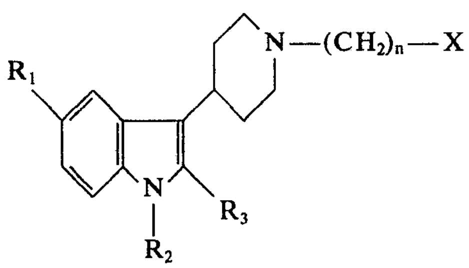

(X)

dans laquelle $R_1$, $R_2$, $R_3$, n et X possèdent la signification indiquée plus haut, au moyen d'une benzimidazolone de formule générale:

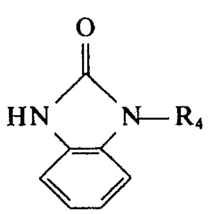

(XI)

dans laquelle $R_4$ possède la signification indiquée plus haut, et en ce qu'éventuellement on transforme un produit final de formule générale I préparé selon l'un des procédés A à D, de manière en soi connue, en un sel d'addition d'acide physiologiquement supportable.